# EUROPEAN PATENT APPLICATION

(11) **EP 4 751 643 A1**
(43) Date of publication of application: **03.06.2026**
(21) Application number: 24215730.3
(22) Date of filing: 27.11.2024
(51) Int. Cl.: A61B 5/05, A61B 5/00

(54) **DYNAMIC ESTIMATION OF LANDING POSITION FOR PROXIMAL END OF STENT**

(71) Applicant: Koninklijke Philips N.V., 5656 AE Eindhoven (NL)
(72) Inventor: SINHA, Ayushi, Eindhoven (NL); FOTOUHI, Javad, Eindhoven (NL); LEE, Brian Curtis, 5656AG Eindhoven (NL); FEIZPOUR, Amin, Eindhoven (NL)
(74) Representative: Philips Intellectual Property & Standards

(57) **Abstract**

The present invention relates to a device and method for assisting in placement of, e.g., an endovascular device for treatment of an aneurism comprising a data processor configured to receive, e.g., image data representing an anatomical region of interest including a vasculature, and at least a distal portion of a placement device together with the endovascular device located in the vasculature. The data processor or controller is configured to interpret the image data, to estimate a proximal landing position of a proximal end of the endovascular device and to determine instructions for positioning and deployment of the endovascular device while the endovascular device is in engagement with an inner shaft of the placement device.

## Description

### FIELD OF THE INVENTION

The present invention relates to minimally invasive and image-guided procedures for deploying stent-like devices for medical therapy. In a particular embodiment, the present invention relates to devices and methods for controlling the positioning and deployment of, e.g., a flow-diverter which can be placed across an aneurysm to redirect blood flow away from the aneurysm.

### BACKGROUND OF THE INVENTION

Stent-like deployment is used to perform a medical therapy of a vascular disease or damage (e.g. local aneurysm, narrowed vessels due to clots, calcification..., vascular lesion ...).

For instance, flow diversion is an increasingly popular endovascular method of treating cerebral aneurysms, potentially due to improved aneurysm occlusion with no increase in complication rates. The deployment of the flow diverter device is a complex maneuver that requires a number of multiaxial endovascular device movements to ensure that the flow diverter mesh is properly positioned and achieves a well-apposed placement. This is a high-stakes maneuver because the flow diverter cannot be recaptured once deployed and correcting a poor flow diverter placement is challenging if not impossible, likely requiring placement of a second flow diverter. Additionally, flow diverter devices are costly. For these reasons, physicians, hospitals, and patients are all incentivized to produce successful flow diversion on the first try.

The quality of flow diverter (or any other type of stent-like device) deployment is generally defined by the accuracy of the distal and proximal ends of the implant as well as the apposition of the flow diverter mesh against the parent vessel wall. The purpose of the implant is to place a mesh over the aneurysm neck (or start of any other type of vascular disease or damage) which serves as a bed for endothelialization and eventual occlusion of the aneurysm (or other type of vascular disease or damage). The quality of endothelialization and occlusion depends on the apposition of the mesh to the vessel wall - the mesh should be well apposed in order to fully occlude the aneurysm. The placement quality of the distal and proximal ends of the implant is measured by the coverage of the aneurysm, placement in a location that ensures good anchoring of the device, and placement in a location that avoids occlusion of secondary blood vessels branching off of the parent vessel. In practice, only the distal end's placement can be finely controlled. The proximal end's eventual location is determined partially by the device length and partially by the degree of packing of the mesh during placement.

During flow diverter or stent deployment, the final proximal landing position achieved when the deployment is completed has an impact on its effectiveness. Suboptimal positioning of the proximal end of the flow diverter may prevent appropriate endothelialization for successful blocking of blood flow to the aneurysm or may block bifurcating branches in the vessel preventing blood flow to critical parts of the brain. The known length of a flow diverter is not sufficient information to predict its proximal landing position due to foreshortening.

US 2023/0190225 A1 describes a system including a processor circuit that receives intraluminal images of a body lumen. The processor circuit identifies a stent edge within the intraluminal images and calculates a distance between the stent edge and the vessel wall. The processor circuit additionally compares the distance between the stent edge and the vessel for the intraluminal images to a threshold distance and identifies which intraluminal images correspond to a distance exceeding the threshold. The processor circuit further outputs to a display a longitudinal view of the body lumen identifying locations along the stent at which the distance between the stent and the vessel wall exceeds the threshold distance.

The effective placement of endovascular devices is contingent upon accurately determining their location and shape. This implies that the precise positioning of the device in relation to proximal and distal landing targets, along with ensuring proper vessel wall apposition, are critical for the overall success of the procedure.

### SUMMARY OF THE INVENTION

Therefore, there is a need to accurately estimate where a point, e.g., at the proximal end of the flow diverter will land and dynamically update this estimate throughout the deployment to allow users or machine to adjust the positioning of the flow diverter early and avoid downstream complications. Furthermore, there may be a need to provide a controller which assesses and tracks various parameters and features linked to device deployment during the deployment, and if any of these parameters exceed their threshold, the controller generates an output command. This output command may be employed to adjust the device's position both in terms of direction and velocity.

The object of the present invention is solved by the subject-matter of the independent claims; further embodiments are incorporated in the dependent claims. This invention offers the capability to extract device features from, e.g., images and estimate the optimal device movement, estimate final placement during deployment, ensuring its location, direction and/or shape align appropriately with the anatomy. Additionally, a controller can automate the positioning and deployment of devices when integrated with a robotic system.

The controller or data processor outlined in this disclosure has the potential to enhance standardization, increase precision and deployment accuracy, leading to improved care and reduced complications.

It should be noted that the following described aspects of the invention apply also for the system for assisting in placement of a device like an endovascular device, for the corresponding method which may be implemented as computer program product, and for the non-transitory computer readable medium having stored such a computer program product.

In general, a system for assisting in deployment of a device to be deployed for medical therapy is provided, wherein the device comprises a mesh structure allowing deployment of the device from a collapsed configuration to a deployed configuration, wherein the mesh structure has a shape which changes during deployment resulting in a change of a length of the device. The system comprises a data processor configured to receive data, wherein the data relate to a position of a first point of the device and to the change of the length of the device, the device being at least partially in the deployed configuration. The data processor is configured to estimate a position of a second point of the device after deployment, based on (i) the received data related to the position of the first point of the device, and (ii) the received data related to the change of the length of the device.

According to an embodiment, the device is an endovascular device and the received data are related to a vasculature, wherein the position of the first point is identified in relation to the vasculature, and wherein the position of the second point is estimated additionally based on the data related to the vasculature.

As disclosed herein, a system for assisting in placement of an endovascular device to be deployed for medical therapy, may comprise a data processor configured to receive image data, to interpret the image data and to estimate a position of the ends of the endovascular device after deployment. The endovascular device may comprise a mesh structure allowing deployment of the endovascular device from a collapsed configuration for delivery to a deployed configuration. In particular, the mesh structure may have a length which changes during deployment.

It is noted that the device which may deployed for medical therapy may be an endovascular device like a stent-like device, a flow-diverter with a mesh-type structure, an intrasaccular device, an intra-cranial stent, but is not limited to such devices. The vasculature encompasses arterial and venous vessels as well as deformations of vessel walls like aneurisms. While a stent-like device may be placed in a vessel so as to open up a calcified portion thereof, a flow-diverter may be placed in a vessel so as to bridge or treat an aneurism, and a woven endobridge device may be placed in an aneurism so as to fill the same. In this disclosure, some aspects are described in the context of an intra-cranial flow diverter as an example providing an understanding of the invention.

The image data as received by the data processor represent an anatomical region of interest including a vasculature, and at least a distal portion of a flexible elongated placement device together with the endovascular device located in the vasculature. The endovascular device may at least be partially accommodated within the distal portion of the placement device. The received image data may be X-ray image data which may be generated, e.g., by an intra-operative C-arm based X-ray imaging device or the like. The anatomical region may be enhanced by a contrast agent in the X-ray image, i.e. the image data may represent an angiography image or a roadmap image (live X-ray image overlaid on an angiography image). Other devices for generating X-ray image data can be used. The image data may include at least one out of the group consisting of X-ray projection image data, a 3D reconstruction based on a plurality of X-ray projection images generated from different directions, a 3D computer tomography (CT) scan, a 3D cone beam CT scan, a 3D magnet resonance tomography scan, a 3D model of at least part of the anatomical region of interest, and a combination thereof. Alternatively or additionally, the image data may be processed utilizing artificial intelligence.

The placement device which may be used for deployment of the endovascular device, comprises a distal end, a proximal end, a longitudinal direction extending between the distal end and the proximal end, an outer sheath extending along the longitudinal direction, and an inner shaft extending along the longitudinal direction, wherein the outer sheath and/or the inner shaft are movable one to the other in the longitudinal direction of the placement device. In other words, the outer sheath is movable in the longitudinal direction of the placement device and/or the inner shaft is movable in the longitudinal direction of the placement device. The inner shaft may be a wire like a guidewire. The outer sheath may be a catheter. An endovascular device, like those mentioned above, may be placed in the vicinity of the distal end of the placement device between the inner shaft and the outer sheath, for introduction of the endovascular device into the vasculature and to the intended placement site. The movements of the inner shaft and the outer sheath relative to each other may be supported and/or fully controlled by a robotic device.

According to an embodiment, the endovascular device may comprise an engagement feature for engagement at the distal end of the inner shaft of the placement device. It is noted that an engagement feature may be configured to block any movement of the endovascular device in a longitudinal direction when the endovascular device is compressed and placed between the inner shaft and the outer sheath of the placement device. The blocking of longitudinal movements is in particular of advantage for a partial deployment of the endovascular device, when the outer sheath is retracted to some extent but not fully retracted (and/or when the inner sheath is advanced to some extent but not fully advanced). The outer sheath is fully retracted (and/or the inner sheath is fully advanced) when the endovascular device can expand so that the engagement feature will disengage from the inner shaft of the placement device by a movement in a radial direction away from the inner shaft. In the context of this disclosure, retracting the outer sheath (and/or advancing the inner shaft) and allowing the endovascular device to expand is denoted as "unsheathing". It is noted that the engagement feature allows for pulling the endovascular device into the outer sheath of the placement device by either pulling the inner shaft proximally or by pushing the outer sheath distally. Bringing the endovascular device back into the outer sheath is denoted herein as "resheathing".

The data processor (or controller) is configured to interpret the image data and to determine instructions for positioning and deployment of the endovascular device while the endovascular device is in engagement with the placement device. The interpretation of the image data may include at least an identification of the position of a first point (e.g. the distal end) of the endovascular device relative to the vasculature and an identification of a vasculature wall defining a vasculature geometry. Based on these two identified aspects, a position of a second point (e.g. the proximal end) of the endovascular device after deployment can be estimated, when further taking into account the change of the length of the mesh structure when expanding the endovascular device from a configuration when being at least partially accommodated within the distal portion of the placement device to the deployed configuration within the vasculature geometry.

It is noted that the first point may be at a distal end of the endovascular device and the second point may be at a proximal end of the endovascular device. In the context of this disclosure, the term "end" is intended to mean at least a point at or in the vicinity of an end of a device or at least a point in a portion of the device being close to and including the end of the device. The term "end" may be specified as "first end", "second end", "distal end" or "proximal end". With the term "end" meaning at least a point, also the terms "first point" and "second point" are used herein.

According to an embodiment, interpreting the image data may include an identification of a wall apposition at the vascular wall of the part of the endovascular device being not accommodated within the placement device. Based on the wall apposition of the already deployed part of the endovascular device at the vascular wall, the position of the proximal end of the endovascular device after deployment may be estimated with improved accuracy.

A further improvement may be achieved when estimating the position of the proximal end of the endovascular device after deployment is further based on data from the robotic device controlling the movements of the inner shaft and the outer sheath relative to each other. Data from robotic device may be available from sensors on robotic components controlling movement of devices, from controllers operated by a user, and/or from automated control decisions computed by a computer. These movements may determine whether an unsheathing or a resheathing or a change in the diameter of the endovascular device is being performed, which contribute to the estimate of the position of the proximal end of the endovascular device after deployment.

A further improvement may be achieved when estimating the position of the proximal end of the endovascular device after deployment is further based on the material properties of the endovascular device, the material properties include elasticity, pretension and/or stiffness. It may be noted that the material properties may vary when considering the properties in longitudinal or radial directions. For example, the mesh structure of the endovascular device may be stiffer in longitudinal direction than in radial direction allowing for a radial expansion with less change of the length.

The above-mentioned interpretation of the image data may be based on predetermined thresholds, wherein the thresholds may vary based on factors such as the type of the device, anatomical characteristics, device(s) distance to the distal target and the proximal target. The position of the endovascular device may be identified in a longitudinal direction of the vasculature.

When the estimated position of the second point or end, e.g., the proximal end of the endovascular device after deployment is inside a predetermined area of the vasculature, the data processor may be configured to determine instructions for positioning and deployment of the endovascular device including determining instructions for movement of the outer sheath and for movement of the inner shaft so that the endovascular device is positioned at a predetermined position in the vasculature and is deployed with a predetermined vasculature wall apposition.

The determined instructions for positioning and deployment of the endovascular device may include instructions of (i) moving the inner shaft together with the outer sheath by pushing the inner shaft and the outer sheath distally so that the position of the endovascular device is changed relative to the vasculature, (ii) moving the inner shaft together with the outer sheath by pulling the inner shaft and the outer sheath proximally so that the position of the endovascular device is changed relative to the vasculature, (iii) moving the inner shaft together with the outer sheath by pushing the inner shaft and the outer sheath distally so that the diameter of at least a portion of the endovascular device is increased for vasculature wall apposition, (iv) moving the outer sheath relative to the inner shaft so that the endovascular device is unsheathed, and (v) moving the outer sheath relative to the inner shaft so that the endovascular device is resheathed. It is noted that a combination of the mentioned movements is also envisaged. For example, the inner shaft may be pushed distally to a greater extent as the outer shaft, resulting in an improvement of the apposition at the vasculature wall and an unsheathing at the same time. This example reflects a combination of the movements mentioned above under (iii) and (iv). Other combinations may be applied for advantage. Summarizing, the inner shaft and the outer sheath can be moved independent from each other and any combination of movements of the inner shaft and the outer sheath may be appropriate so as to achieve both the intended position of the endovascular device relative to the longitudinal direction of the vasculature and the sufficient apposition at the vasculature wall. As will be understood by a skilled person, further elements may be provided working together with either or both of the inner shaft and the outer sheath so as to achieve the unsheathing or resheathing.

The determined instructions for positioning and deploying of the endovascular device may be evaluated by estimating the position of the proximal end of the endovascular device after deployment based on these determined instructions. Accordingly, it is possible to iteratively improve the deployment and positioning of the endovascular device by, firstly, estimate the landing position of the proximal end of the endovascular device, secondly, determine next deployment actions, and thirdly, evaluate the estimated landing position based on the determined deployment actions. Those steps may be performed after each actually performed deployment or positioning step, or after some performed steps. In other words, the deployment and positioning of the endovascular device may be fine-tuned.

The data processor is typically configured to provide an output representing the determined instructions for positioning and deployment. The output may include a visualization of instructions for moving of the inner shaft and of the outer sheath on a display device so that a user may be guided when deploying an endovascular device in a vasculature. The display may be a monitor or a head mounted display allowing for an augmented visualization and/or a 3D view on the arrangement of the outer sheath and the inner shaft together with the instructions. For example, the output may indicate by arrows whether the outer sheath and/or the inner shaft shall be moved distally or proximally. Besides being visible, the output may also be textual or audible, but not limited to. The placement device may consequently comprise a handle with manual actuation elements engaging the proximal end of the inner shaft and engaging the proximal end of the outer sheath, so that the inner shaft and the outer sheath can be manually moved in accordance with the determined instructions.

Alternatively, and/or additionally, the output representing the determined instructions for positioning and deployment may include instructions executable by a robotic device performing the movements of the inner shaft and the movements of the outer sheath. In consequence, the robotic device may comprise a first feature engaging the proximal end of the inner shaft and a second feature engaging the proximal end of the outer sheath so that the inner shaft and the outer sheath are moved in accordance with the determined instructions for positioning and deployment of the endovascular device, when the robotic device executes the determined instructions. In other words, the data processor's decision unit transmits a movement signal and the robot controller directs the corresponding movements of the robot arms or end-effectors connected to surgical or endovascular devices.

In accordance with an embodiment, a system for controlling deployment of a mesh-type device like an endovascular device (e.g., a flow-diverter or a deployable stent-like device) in a vascular system, comprises a data processor or controller configured to
- receive a (dynamic) anatomical image including the deployable device,
- determine a deployment phase of the device (e.g. unsheathing, resheathing, loading, unloading for the endovascular device) based on image (and/or robot data if a robot is used to command the deployment and/or positioning)
- evaluate changes in shape and/or position of the endovascular device as it deploys, and
- predict a lengthening or shortening of the position of the device based on the determined deployment phase, preferably using a (trained) model.

According to an aspect, a robot may be provided with commands, or a user may be guided for repositioning of the device or for deployment of the device to compensate said lengthening or shortening.

Further aspects of the present disclosure may be as follows, alone or in combination.

Prior patient data in the form of 3D imaging (e.g., CT, cone beam CT, MRI) may be used in addition to live fluoroscopy imaging in order to provide additional context. Such imaging may serve as a digital twin of the patient. Alternatively, a 3D model of a representative anatomy (e.g., a 3D image from a large data library that is determined to be as close as possible to the current patient anatomy, a 3D atlas representing an average anatomical model) may alternatively be used. In both cases, a registration between 3D imaging or 3D model and live fluoroscopy may be required to ensure that the correct anatomy is used in any computations or estimations. In the case of a 3D model not acquired directly from the patient, a deformable registration may be necessary to deform the 3D model to match the anatomy of the patient as seen in the live fluoroscopy before any estimates based on the 3D model are computed. Artificial intelligence may be utilized for computing the registration (e.g. when providing and/or processing a model of the anatomical region of interest), generating a 3D model of at least part of the anatomical region of interest (e.g., from CT, cone beam CT, etc.), interpreting the image data, estimating the position of the second end of the endovascular device after deployment and/or determining instructions for positioning and deployment of the endovascular device. The data processor may incorporate a controller system that is a model trained based on training data. The model may take the form of a neural network or a classical machine learning model, trained using various techniques such as supervised, self-supervised, unsupervised learning, clustering, reinforcement learning, and more. For any such models, data from prior procedures can be utilized to train this controller model. The data processor may perform complex apposition calculations using a digital twin or other simulation in order to determine optimal loading, unloading, unsheathing, resheathing, anchoring, etc.

The device may not only provide instructions for but may comprise a robotic device, wherein the robotic device comprises a first feature engaging the proximal end of the inner shaft and/or a second feature engaging the proximal end of the outer sheath so that the inner shaft and/or the outer sheath are moved in accordance with the determined instructions for positioning and deployment of the endovascular device, when the robotic device executes the determined instructions. The robotic device may comprise at least one sensor providing information with respect to the amount of movement and the force applied when moving.

The device may be configured to provide an alert when the estimated position of the second end of the endovascular device after deployment is outside of a predetermined area of the vasculature.

The data processor may further incorporate additional auxiliary input related to tension or haptics. This supplementary information can be derived by either a dedicated tension estimator unit or by comparing the proximal movement of the device (e.g., recorded by robot encoders) with the movements observed at the distal end through visual imaging. The additional auxiliary input may also relate to the orientation of the proximal or distal segments of device(s).

The data processor may accommodate devices of known shapes such as shaped wires and catheters, steerable devices (e.g., steerable catheters), and devices with tracked shapes (e.g., fiber optic real-shape sensing catheters). In the case of the latter, the tracking of the shape device's location can serve as auxiliary input to the data processor.

Further, the data processor may be configured to identify instances where successful deployment cannot be achieved prior to stent release. In response, the data processor may initiate actions such as full or partial resheathing or a complete change of the endovascular device.

The data processor may also examine both the distal and anticipated proximal segments of, e.g., a flow-diverter. Subsequently, it may issue pertinent commands to ensure a safe margin is maintained relative to the aneurysm. The data processor may verify whether the endovascular device is obstructing any perforators or side vessels. It may then adapt the output commands to prevent or rectify such obstructions.

The data processor may involve apposition measurement and control determined by the shape of the device and vasculature in 2D and/or 3D. The shape of the device or parent vessel can be established through segmentation, contour extraction, or key-point identification.

The data processor may be designed so that when the endovascular device is unsheathed until a point-of-no-return, it can detect this event and resheath the endovascular device if necessary. Additionally, if the controller is connected to a robot, it has the capability to temporarily halt the movement until the shape and location of the endovascular device are confirmed. Additionally, the controller may also evaluate the likelihood of successful endovascular device placement and only halts the movement at the point-of-no-return if the likelihood of successful endovascular device placement is lower than some predetermined threshold.

The data processor may support the placement of not only a single endovascular device but also multiple stacked or telescoped endovascular devices. The data processor may, e.g., process information including the shape of the first (or previous deployed) endovascular device, malapposition at the proximal end of the first (or previous deployed) endovascular device, shape of the anatomy near the proximal end, etc. to determine the distal end of the next endovascular device to be deployed such that the stacked deployment will improve the apposition at the proximal end of the first (or previous deployed) endovascular device. The data processor may also determine the size of the next endovascular device to be deployed. Furthermore, it facilitates ballooning the endovascular device or adjusting the movement of the micro-catheter both forward and backward, enhancing its own apposition as well as the apposition of the first (or previous deployed) endovascular device post-deployment.

The data processor may accommodate additional coordinated movements, including maneuvers designed for untangling or detaching processes.

Another aspect of the disclosure is a method for assisting in placement of an endovascular device. The method generally comprises the steps of receiving image data, interpreting the image data, and estimating a position of a second end of the endovascular device after deployment.

As outlined above, the image data represent an anatomical region of interest including a vasculature, and at least a distal portion of a flexible elongated placement device together with the endovascular device located in the vasculature, wherein the endovascular device is at least partially accommodated within the distal portion of the placement device and comprises a first end, a second end and a mesh structure allowing deployment of the endovascular device from a collapsed configuration for delivery to a deployed configuration, wherein the mesh structure has a length which changes during deployment, and wherein the placement device comprises a distal end, a proximal end, a longitudinal direction extending between the distal end and the proximal end, an outer sheath extending along the longitudinal direction, and an inner shaft extending along the longitudinal direction, wherein the outer sheath and/or the inner shaft are movable one to the other in the longitudinal direction of the placement device.

The step of interpreting of the image data may include an identification of the position of a first point or end of the endovascular device relative to the vasculature, an identification of a vasculature wall defining a vasculature geometry, and optionally an identification of a wall apposition at the vascular wall of the part of the endovascular device being not accommodated within the placement device.

The step of estimating a position of the second point or end of the endovascular device after deployment may be based on the identified position of the first point or end of the endovascular device relative to the vasculature, on the vasculature geometry, and on a change of the length of the mesh structure when the endovascular device expands from a configuration when being at least partially accommodated within the distal portion of the placement device to the deployed configuration within the vasculature geometry. Further, the step of estimating may be based on the wall apposition of the already deployed part of the endovascular device at the vascular wall.

In accordance with an embodiment, the method may comprise the step of determining instructions for positioning and deployment of the endovascular device including determining instructions for movement of the outer sheath and for movement of the inner shaft so that the endovascular device is positioned at a predetermined position in the vasculature and is deployed with a predetermined vasculature wall apposition. Such determined instructions may be taken into account when estimating the position of the second end of the endovascular device after deployment and may, additionally or alternatively, be provided to a robotic device which is thus caused to move the inner shaft and the outer sheath.

When the estimated position of the second point or end of the endovascular device after deployment is outside of a predetermined area of the vasculature the method may comprise the step of providing an alert.

According to a further aspect of the disclosure, a computer program is provided implementing the steps of the method for assisting in placement of an endovascular device. In particular, the interpretation of received images and the determination of instructions suitable for achieving the reliable position within a vasculature and apposition at the vasculature wall may be implemented as computer program.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows a deployment controller with inputs and outputs.
Fig. 2 shows a micro-catheter introduced into a vessel.
Fig. 3 shows a micro-catheter together with a flow-diverter positioned in a vessel for deployment of the flow-diverter.
Fig. 4 shows an example of a flow-diverter with an insufficient apposition at the vessel wall.
Fig. 5 shows an example of a flow-diverter with inaccurate longitudinal localization within the vessel.
Fig. 6 shows an example of a flow-diverter properly deployed in a vessel.
Fig. 7 is a flow chart of a method of controlling a deployment of a flow-diverter.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

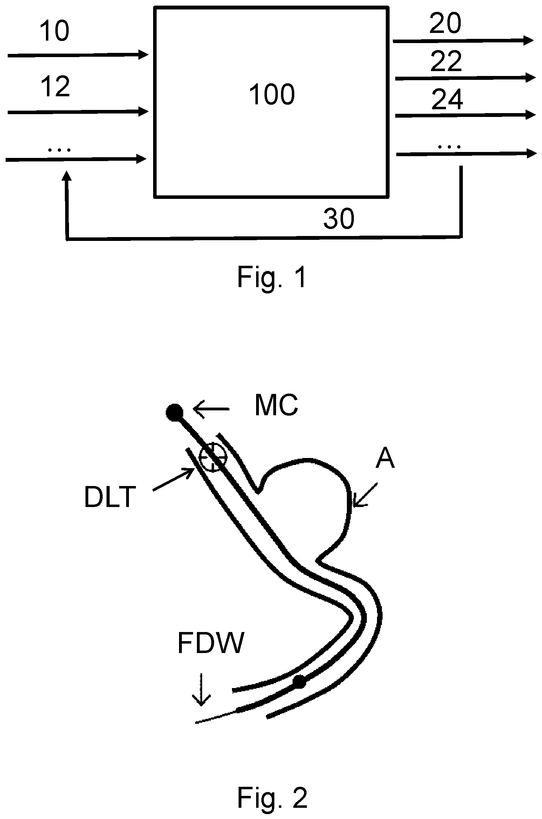

Fig. 1 schematically shows a deployment controller 100 designed to utilize imaging data acquired from stent-like devices for extracting device features. In Fig. 1, such input features are denoted as 10, 12, ... These features are then employed to identify optimal movements for deployment. The core element lies in the decision-making capabilities of the controller 100, which facilitates optimal movements of stent-like devices by considering imaging data, imaging parameters, robot data, and other auxiliary inputs. An auxiliary input may be, for example, the location of the proximal end of the endovascular device as estimated by the controller. As indicated by arrow 30 in Fig. 1, the estimated position of the proximal end of the endovascular device after deployment is an output of the controller which may, then, be an input when determining the next step in deployment and/or positioning of the endovascular device.

It is noted that the controller 100 may also be denoted as data processor or may comprise a data processor providing the functionality as described in this disclosure.

In the context of a flow-diverter (but may be applicable to any other type of endovascular device deployable to perform a medical therapy of a vessel), the output movements encompass a range of actions, including but not limited to loading, unloading, unsheathing, resheathing, anchoring, and moving to the target. Such output movements are denoted in Fig. 1 as 20, 22, 24, ... The devices under consideration consist of, but are not restricted to, stents, flow-diverters, intrasaccular devices, and stent-retrievers. The execution of output commands may necessitate the movement of a single device (e.g., a flow-diverter) or the coordinated movement of multiple devices (e.g., a micro-catheter and a flow-diverter).

This system may utilize 2D imaging data, including fluoroscopy images, roadmap images, digitally subtracted images, etc., and/or 3D images such as 3D rotational angiographies or cone-beam CT scans as input 10, 12, .... Additional auxiliary inputs may also be incorporated, encompassing but not limited to device shapes, device characteristics information, pre- or intra-operative plans, anatomical information, and robotic data.

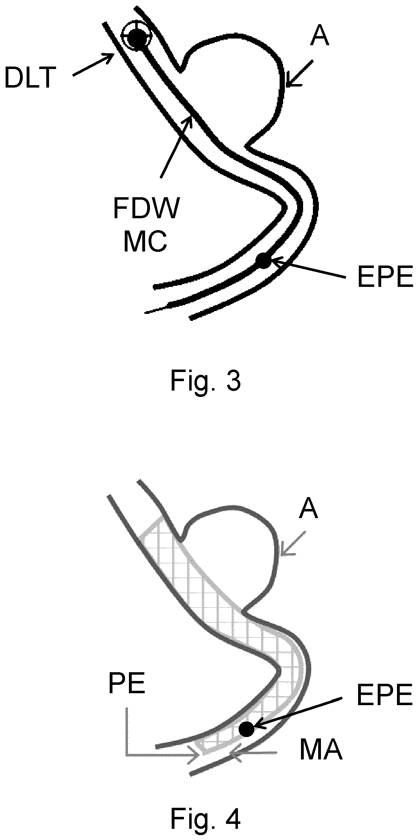

The deployment controller 100 can be assembled from either a single or multiple sub-control systems. The controller may be employed to navigate the devices to the treatment site. A device feature extraction module extracts crucial features, such as the distance between the tip of the microcatheter and the delivery wire to a landing target. Using these features, appropriate commands are generated to maneuver the devices to the target. Exemplary commands include pull-back on the microcatheter and push-forward on the delivery wire. The target location can be automatically determined by the feature extraction module or manually specified by the user. Fig. 2 and 3 show examples of microcatheters and flow-diverter delivery wires moved so as to be at the distal landing target.

Fig. 2 is a schematic illustration of a vessel with an aneurysm A. Within the vessel, a micro catheter MC is placed and a flow-diverter delivery wire FDW is shown in a proximal section of the micro catheter MC. The distal end of the micro-catheter MC is shown with an enlarged head, although the micro-catheter MC may also be provided without such a head and, instead, with a continuous thickness at its distal end. The flow-diverter delivery wire FDW is configured to move like an inner shaft within the micro-catheter MC which may be considered as an outer sheath surrounding the inner flow-diverter delivery wire FDW. Further indicated in Fig. 2 is a designated landing target DLT. Fig. 2 can be seen as illustrating a state at the beginning of a procedure, i.e. before a placement of an endovascular device in a vessel.

Fig. 3 illustrates the vessel with the aneurysm A as in Fig. 2. Fig. 3 also shows the designated landing target DLT. As may be considered as a state initial to a deployment of a flow-diverter, both the flow-diverter delivery wire FDW and the micro-catheter MC are placed with their respective distal end located at the designated landing target DLT. With the distal end of the flow-diverter at the designated landing target DLT, the controller may estimate the position of the proximal end assuming that the flow-diverter will be deployed with an appropriate fit in the vasculature, i.e., with an appropriate apposition of the mesh structure of the flow-diverter at the vascular wall. The estimated proximal end is denoted in the figure with EPE. As will be understood, the "distal end" and the "proximal end" (as mentioned herein) are examples of a first point and of a second point a position of which is determined and/or estimated by the system.

While it is appreciated that the movements of the inner shaft and the outer sheath may be performed manually, a robotic device may also control those movements. According to this disclosure information from a robot controller, an image processing controller, and an information processing controller may be combined to dynamically estimate the proximal landing position of a flow diverter during deployment.

The robot controller may enable the determination of the deployment phase. Deployment of a flow diverter consists of several types of movements of the microcatheter MC housing the flow diverter and the delivery wire of the flow diverter FDW, performed to achieve a successful deployment. These movements include:
Unsheathing, where the microcatheter is pulled back to allow the flow diverter mesh to expand
Resheathing, where the microcatheter is pushed forward to recapture the flow diverter mesh
Loading, where the microcatheter and the delivery wire are pushed forward together to force the flow diverter mesh to open up and adhere to the vessel walls better
Unloading, where the microcatheter and the delivery wire are pulled back together to remove tension build up in the system
Each of these movements changes the proximal landing position of the flow diverter. The robot controller can determine the movements being performed or phase of deployment via the actions being performed by the robot arms or robot controllers controlling the microcatheter MC and the delivery wire FDW. In phases where the flow diverter foreshortens, the proximal landing position moves closer to the distal landing position and in phases where the flow diverter lengthens, the proximal landing position moves away from the distal landing position without exceeding the flow diverter's original length.

While knowing the deployment phase determines whether the flow diverter is foreshortening or lengthening, it does not determine the amount of foreshortening or lengthening because of slack or tension in the system, which results in some but not all of the motion at the actuator translating to the device being deployed within patient anatomy.

The image processing controller enables the determination of the amount of foreshortening or lengthening. Flow diverters are deployed under X-ray image guidance and the image processing controller receives these X-ray images. The X-ray images are processed to identify the flow diverter including its mesh body and landmarks such as the distal end, proximal end, and the point of no return as well as anatomical landmarks such as the vascular structure where the flow diverter will be placed. Tracking these over time (i.e., through multiple frames) allows for the determination of how the flow diverter mesh is changing and the limits (e.g., vessel width) within which the flow diverter can change, as well as how the landmarks are moving.

Finally, the information processing controller receives information about the flow diverter such as the size of the flow diverter including its length and width. These also help determine the limits within which the flow diverter shape can change during the deployment (e.g., flow diverter cannot expand beyond the width of the vessel as well as beyond the maximum width allowable by the flow diverter mesh).

The controller 100 may include the above-mentioned robot controller, image processing controller, and information processing controller and may, during deployment of the flow-diverter, analyze the image and extract features, such as the area and extent of malapposition. This module delineates the contour around the stent and assesses its overlap with the aneurysm's parent vessel. The uncovered area by the stent indicates the degree of malapposition. Whenever malapposition surpasses a threshold, the apposition controller generates commands to initiate system loading. Loading leads to stent expansion, enhancing vessel wall apposition. An exemplary loading command involves simultaneous push-forward on both the micro-catheter and the flow-diverter. When tension in the system builds up, the system can send command to unload the system by simultaneous pull-back on the micro-catheter and the flow-diverter.

Fig. 4 illustrates a situation with a flow-diverter being deployed within the vessel but with a malapposition MA at its proximal end portion. As can be seen in Fig.4, the estimated proximal end EPE of the flow-diverter is not at the location of the actual proximal end PE of the flow-diverter. In the illustrated situation, the mesh structure is not sufficiently expanded causing the malapposition and, consequently, the mismatch of the deployed proximal end PE with the estimated proximal end EPE estimated at the start of or during deployment. In an ideal deployment, the controller may assume that the flow-diverter will be further expanded which will result in a foreshortening of the flow-diverter so that the estimated proximal end EPE of the flow-diverter will be closer to the distal end of the flow-diverter then the actual proximal end PE.

Another sub-controller may ensure the flow-diverter opens at the designated landing target DLT. This controller analyzes the image during the unsheathing or deployment process of the flow-diverter. As an example, as the stent opens it may gently propel the flow-diverter stent upward, preventing it from dislodging from the designated landing target. Once the flow-diverter opens and anchors to the vessel walls, further upward push ceases to avoid excessive tension. In another example, during the anchoring phase, the distal end of the flow diverter FD may constantly move towards the desired distal landing target regardless of the current microcatheter movement. In another example, the controller may provide instructions to partially open the flow diverter mesh distal to the distal landing target DLT and move the delivery wire FDW and micro-catheter MC as a linked group until the flow diverter's distal end is in the desired position DLT, then perform the deployment until anchoring is achieved. That is, throughout the deployment, it continually monitors the distal landing target. If the distance between the flow diverter distal end and the desired distal landing target exceeds a predetermined threshold, the controller generates commands to realign the flow-diverter with the distal landing target. The controller is also equipped to issue resheathing commands if the flow-diverter's location necessitates resheathing after anchoring has occurred.

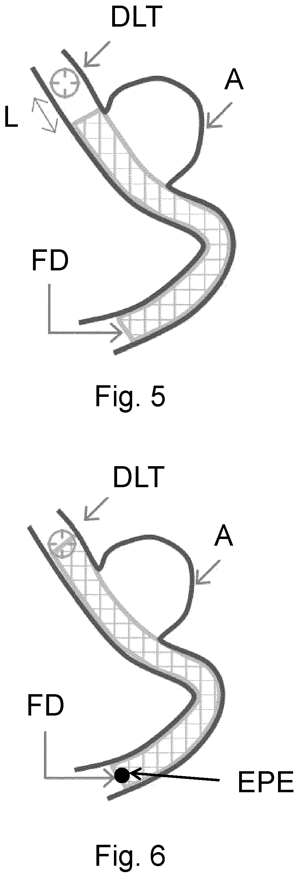

While Fig. 5 illustrates a flow-diverter FD positioned in a vessel with its distal end being away from the designated landing target DLT at a distance L, Fig. 6 shows the flow-diverter FD with its distal end being accurately positioned at the designated landing target DLT. The positioning of the flow-diverter FD as in Fig. 6 ensures that blood flow through the vessel will be diverter away from the aneurysm A. After fully deploying the flow-diverter, the estimated proximal end EPE of the flow-diverter should be at the same location as the actual proximal end of the flow-diverter FD.

In the following, steps of an exemplary method are described for further understanding of the present disclosure. It will be understood that the steps described are major steps, wherein these major steps might be differentiated or divided into several sub-steps. Furthermore, there might be also sub-steps between these major steps. It will also be understood that only part of the whole method may constitute the invention, i.e. steps may be omitted.

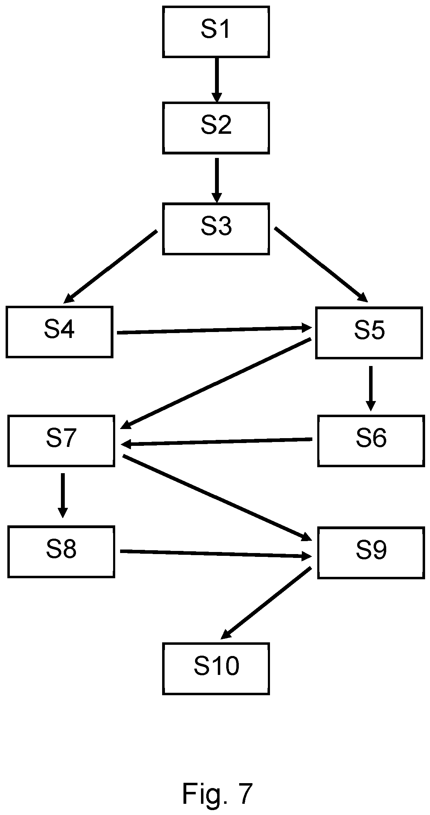

Steps for the deployment of a flow-diverter are schematically depicted in the flow-chart of Fig. 7. The initial decision made by the controller involves navigating the micro-catheter distal to the aneurysm (step S 1). Subsequently, the tip of the micro-catheter and the distal end of the flow-diverter are aligned with the distal landing target (step S2).

Following this, in one example, the micro-catheter may be retracted to unsheathe the flow-diverter (step S3). It may be noted that, depending on the type of flow diverter employed, other actions may be performed to unsheathe the flow diverter, such as pushing on the delivery wire. When starting the deployment of the flow-diverter, the controller may repeatedly estimate the proximal landing position of the proximal end of the flow-diverter and consider whether that proximal landing position is within a predetermined area. The controller, depending on whether the flow-diverter is open and securely anchored to the vessel wall, determines whether to move the flow-diverter or not. If the distal part of the flow-diverter is open, the system will automatically cease issuing push-forward commands for the flow-diverter (step S4).

While the micro-catheter undergoes unsheathing, the deployment controller consistently assesses vessel wall apposition by analyzing the device's shape and comparing it to the parent vessel (step S5). It identifies any gaps and determines a malapposition measure based on the size of these gaps (step S6). If the malapposition measure surpasses the loading threshold, the controller will automatically issue the loading command to expand the flow-diverter (step S7). The deployment controller may also consider the proximity of the aneurysm neck or how the packing density will affect the desired proximal landing zone (step S8). With each of steps S5 to S8, the controller may additionally estimate the proximal landing position of the proximal end of the flow-diverter and may take that estimated proximal position into account for fine-tuning the commands or instructions for movements.

When the flow-diverter reaches the point of no return, the controller will generate output movements to fully deploy the flow-diverter (step S9). Subsequently, the controller will send commands to capture the wire and retract the entire device assembly (step S10).

It is noted that a model may be trained based on information retrieved from procedures as outlined above, wherein the model may be a statistical model, a machine learning model, or a deep learning model. During training, the model may learn from experienced neuro-interventionalists by processing the above-mentioned data collected during flow diverter deployment. This allows the model to learn to estimate where the final proximal landing position of the flow diverter will be as soon as the deployment begins (i.e., distal end is deployed) and to fine-tune its estimate throughout the deployment given certain robot control inputs, certain imaging cues, and known information about the flow diverter being deployed. The final proximal landing position is simply the proximal landing position that is expected to be achieved when the deployment is complete. Fine-tuning the estimate throughout the deployment is necessary because each deployment is different, each anatomy is different, and each interventionalist might deploy differently. Allowing the model to update its estimate with every unsheathing, loading, unloading, etc. allows the output to be adapted to these differences.

As mentioned before, knowing the proximal landing position early will allow users to resheath the flow diverter and reposition the distal end early such that after deployment, the proximal end of the flow diverter lands in a favorable position (e.g., on a straight portion of the vessel, not blocking any branching vessels).

Applications that can be enabled by knowing the proximal landing position may be the following:
When the microcatheter is past the proximal landing position, the robot can automatically begin the detachment process (e.g., extracting the delivery wire)
Improve estimation of the point of no return by determining a rough location where it should be relative to the proximal landing position since these two landmarks have a fixed distance between them. This can significantly reduce the search space for the point of no return, which is a small landmark, towards the end of the deployment when knowing its location is most important (since the flow diverter cannot be resheathed past the point of no return). This also enables the point of no return detection to be more robust by estimating a rough location that can act as a fall back when this landmark is not visible in images.

According to an embodiment of the disclosure, the method may rely solely on the image processing controller to infer from the changing flow diverter shape whether unsheathing, resheathing, loading, or unloading is being performed along with the amount of foreshortening and lengthening of the flow diverter. This may allow the estimation of the proximal landing position in scenarios without robot information.

According to a further embodiment, the estimation of the proximal landing position is performed directly by a neural network controller which may be included in controller 100. Instead of a separate robot controller, image processing controller, and information processing controller, the neural network may directly infer how changes in the control and image features affect the final proximal landing position. For instance, the neural network may learn the correlation between device movements caused by actuators or a user controlling the microcatheter and delivery wire (e.g., pushing both forward) and the resulting image features (e.g., flow diverter expanding) and associate these features with an amount of foreshortening to predict the proximal landing position. The device information, as before, may provide constraints to these predictions. The neural network may have the following inputs and outputs:
Input: *I_{d}* = image with segmentation or heatmap indicating the distal end of flow diverter, *rₜ* = {*r*_{*t*₁}*, r*_{*t*₂}*,* ...}, *r_{θ} =* {*r*_{*θ*₁}*, r*_{*θ*₂}*,* ... }, (optionally, *I_{fd}* = image with segmentation or heatmap indicating the flow diverter) → *e* → Output: *Iₚ* = image with segmentation or heatmap indicating the estimated proximal landing position of the flow diverter, where *rₜ* and *r_{θ}* are the robot translation and rotation parameters obtained from the robot per arm (shown here for 2 arms, but generalizable to > 2 arms), and *e* is the encoding learned by the trained neural network that enables the output prediction to be made. The optional input of the flow diverter segmentation allows the network to additionally infer from the image (by observing how the flow diverter is changing, e.g., stretching during unsheathe, or expanding during loading) changes in the flow diverter shape and/or length associated with various robot inputs.

In an implementation of an embodiment where the neural network optimizes each prediction based on a previous prediction, an initial prediction or initialization may need to be provided. This may be the current position of the radio-opaque marker identifying the distal end of the flow diverter at the current time. The radio-opaque marker may be identified in the image using image processing as a preprocessing step. At the start of the deployment, the initial prediction may be the position of the distal end of the flow diverter before any part of the flow diverter has deployed, as shown in Fig. 3. Without any additional information, this is the estimated proximal landing position EPE at the start of the deployment procedure. This information may be input back into the neural network so that the next updated proximal landing position is estimated relative to the current estimate. Such an implementation predicts outputs that change significantly as the users' inputs change. That is, if the user is only unsheathing (e.g., at the start of the deployment), without any additional information on how this user might load or unload the flow diverter, the network predicts an EPE reflecting where the proximal end might land if the entire deployment is performed with unsheathing only (since this is the only information available to the network so far). As soon as the user loads or unloads the flow diverter, the EPE moves closer to the distal end of the flow diverter, reflecting the foreshortening of the flow diverter caused by the loading. However, both this initialization and the input of the current estimated proximal landing position are optional. An absolute estimate of the proximal landing position can also be made, in which case an initialization is not required, and the output does not need to be input back into the neural network. Such an implementation predicts outputs that change slightly as the users' inputs change. That is, the model uses learnings from all its training data to estimate where the EPE will be at the end of the deployment, assuming that the deployment will include all expected actions including unsheathing, loading, unloading, etc. even if the model has not seen the user provide all of these inputs yet. As the user continues deployment, the network's outputs change slightly to reflect the user's personal deployment style which may differ from the training data.

In either implementation, as soon as deployment begins, a new estimation of the proximal landing position can be made based on the various movements deployed in the deployment so far. With each time step, the estimate fine-tunes and converges on an accurate proximal landing position, with the proximal end of the deployed flow diverter coinciding with the estimated proximal landing position as deployment gets closer to completion. When the microcatheter is at the point of no return, the estimated proximal landing position is very close to the actual proximal landing position, allowing the user to evaluate whether this position is acceptable and, if not, to resheath the flow diverter and redeploy at a slightly different distal position.

As will be understood, training data for the neural network can be gathered from previous robotically deployed flow diverters. Data from these deployments may be annotated to show the distal landing position and the final proximal landing position, and robot and image data may be aligned so that changes in the flow diverter and proximal landing position (as seen in the imaging data) are aligned with the robot inputs that caused those changes. During supervised training, the EPE may be compared with the annotated proximal landing position to compute a loss function. The value of the loss function may be computed using functions such as the negative log-likelihood loss, the mean absolute error (or L1 norm), the mean squared error, the root mean squared error (or L2 norm), the Huber loss, or the (binary) cross entropy loss.

If an annotation of the proximal landing position is unavailable, then a model may be trained in an unsupervised or self-supervised fashion:
Input: *I*_{*t,t*+*n*} = image(s) with flow diverter in current state, *r*_{*t,t*+*n*} = {*r*_{*t*₁}*, r*_{*t*₂}*,* ..., *r*_{*t*+*n*₁}*, r*_{*t*+*n*₂}*,* ...}, *r*_{*θ,θ*+*n*} = {*r*_{*θ*₁}*, r*_{*θ*₂}*, ... , r*_{*θ+n*₁}*, r*_{*θ+n*₂}*,* ... }, → *e* → Output: *I*_{*t+n*+1*,t*+*n*+*x*} = image(s) with flow diverter in future state. The current state may be represented by one image (*n* = 0) or multiple images (*n* > 0) showing the current evolution of the flow diverter given robot states spanning the same duration. Here, the network learns an encoding *e* that generates an image (*x* = 1) or multiple images (*x* > 1) estimating the future state(s) of the flow diverter given the current state(s) from input image and robot data. This network may be an encoder-decoder type network that encodes the input using convolutional and/or pooling layers into a low dimensional encoding, which is then used to decode an output using deconvolution and/or un-pooling layers, forcing the encoding to capture the most important information in the input. For instance, the encoder may learn that when the robot pins the flow diverter delivery wire and pulls back the microcatheter, the flow diverter in the image expands in a champagne glass shape and foreshortens the flow diverter moving the EPE closer to the distal landing position; when the robot pins the delivery wire and pushes the microcatheter forward, the flow diverter is resheathed and the EPE moves back towards its original location before the flow diverter was unsheathed; when the delivery wire and microcatheter are both pushed forward, the flow diverter mesh expands, again foreshortening the flow diverter and moving the EPE closer to the distal landing position; and when the delivery wire and microcatheter are both pulled back together, the flow diverter stretches slightly, slightly moving the EPE back away from the distal landing position. The robot data may be concatenated to a low dimensional representation at some layer in the encoder. As before, the trained network can predict EPE iteratively with the EPE converging to the final location as the flow diverter deployment continues or the trained network may use learnings from full deployments to predict the final EPE throughout the deployment with the prediction fine-tuning slightly as the deployment continues.

During self-supervised training, the output image(s) may be compared with the available images representing the flow diverter in future state(s) allowing the model to learn from real world data. Similar loss functions as those mentioned earlier may be used to compare predicted and known images. Additionally, a contrastive loss may be used to force the encodings to be as separable as possible. For instance, ideally all encodings representing unsheathing would be clustered together in the encoding space but distant from encodings representing resheathing or loading or unloading. This can be done by forcing the distance between encodings of the same state (e.g., unsheathing) to have a small distance, while forcing encodings from different states to have larger distances in encoding space. States can be known from robot state information (i.e., which devices are being pushed, pulled, or pinned). As before, similar loss as mentioned earlier may be used in encoding space. During training, the value of the loss function is typically minimized over several iterations, and training is terminated when the value of the loss function satisfies one or multiple stopping criteria. Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers". These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the loss function. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the loss, or difference between the predicted output data and the ground truth data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data. The trained neural network may output images and/or graphical elements, or a further image processing may process the outputs of the neural network to convert them in images), adapted to be displayed on a user interface (e.g. a screen or other), depicting the estimated / predicted deployment in a near or far future. For instance, the trained neural network may obtain images depicting an at least partially undeployed flow diverter and/or graphical elements showing robot inputs indicating e.g. that the flow diverter delivery wire is pinned (stationary) and/or microcatheter is being pulled back, resulting in the neural network outputting, at least indirectly, an image of a future state depicting a (slightly) more open flow diverter and an EPE that is (slightly) closer (or further) to the distal landing position. In another example, the trained neural network may obtain images of a partially deployed flow diverter and robot inputs indicating that both the delivery wire and microcatheter are being pushed forward, resulting in the neural network outputting an image of a future state depicting a loaded flow diverter with an expanded mesh and an EPE that closer (or further) to the distal landing position.

Summarizing, this disclosure is about a computer-based controller of a deployment of a mesh-type device (e.g. stent, flow-diverter, and stent-retriever, balloon..) in a vessel (or another organic lumen) arranged to get dynamic monitoring data related to the positioning and deployment of said mesh-type device within the vessel, process said monitoring data to generate metrics representative of a state of the device deployment within the vessel, said metrics comprising direction of deployment (with respect to the directions of the adjacent vessel walls) and magnitude (or opening angle) of the mesh deployment, and generate an output signal using said metrics that indicates the estimated landing position of the proximal end of the mesh-type device. Additional output signals may be generated to ensure the proximal end of the mesh-type device lands at the estimated landing position, if the estimated landing position is acceptable by the user. As an example, if said metrics exceed a predetermined threshold, said threshold being predetermined so as to be representative of a too high level of risk for the vessel and/or for the deployment, an alert may be generated.

In a particular embodiment, this output signal is a command to be employed to robotically adjust the device's position both in terms of direction and magnitude. The output command may be movements encompassing a range of actions (e.g. loading, unloading, unsheathing, resheathing, anchoring, moving to the target...).

As an option, the controller is further arranged to get additional auxiliary inputs, encompassing but not limited to device shapes, device characteristics information, pre- or intra-operative plans, anatomical information, an estimated position of a proximal end of the flow-diverter and robotic data, implement said process step based on the additional auxiliary inputs, in addition to the monitoring data.

Optionally said additional auxiliary input includes tension or haptics, and may be derived by either a dedicated tension estimator unit or by comparing the proximal movement of the device (e.g., recorded by robot encoders) with the movements observed at the distal end through visual imaging.

As an option said output signals can change based on factors such as the type of the device, anatomical characteristics, device(s) distance to the distal target, the point of no return, and the proximal target.

Furthermore, the controller may further be arranged to dynamically receive imaging data of said mesh-type device within the vessel, and dynamically extract said monitoring data related to the positioning and deployment of the device from the image features, including an estimation of the landing position of the proximal end of the flow-diverter during deployment.

By this way, the invention lies in a decision-making capabilities of the controller, which facilitates optimal movements of a mesh-(or stent-)like device by optionally considering imaging data, imaging parameters, and other auxiliary inputs.

Optionally, the system comprises a user interface arranged to assign visual, textual, or auditory elements corresponding to the controller output signal.

The controller may further make use of a model trained on data, such as e.g. a neural network or a classical machine learning model, trained using various techniques such as supervised, self-supervised, unsupervised learning, clustering, reinforcement learning, and more. Specifically, in a supervised training model, data from prior procedures can be utilized to train this controller model.

According to an aspect of the present disclosure, a device is provided for assisting in placement of an endovascular device to be deployed for medical therapy, wherein the endovascular device comprises a first end, a second end and a mesh structure allowing deployment of the endovascular device from a collapsed configuration for delivery to a deployed configuration, wherein the mesh structure has a length which changes during deployment,
the device comprising a data processor configured to receive image data, wherein the image data represent an anatomical region of interest including a vasculature, and at least a distal portion of a flexible elongated placement device together with the endovascular device located in the vasculature, the endovascular device being at least partially accommodated within the distal portion of the placement device,
wherein the placement device comprises a distal end, a proximal end, a longitudinal direction extending between the distal end and the proximal end, an outer sheath extending along the longitudinal direction, and an inner shaft extending along the longitudinal direction, wherein the outer sheath and/or the inner shaft are movable one to the other in the longitudinal direction of the placement device,
wherein the data processor is configured to interpret the image data including an identification of the position of the first end of the endovascular device relative to the vasculature and an identification of a vasculature wall defining a vasculature geometry, and
wherein the data processor is configured to estimate a position of the second end of the endovascular device after deployment, based on
   (i) the identified position of the first end of the endovascular device relative to the vasculature,
   (ii) the vasculature geometry, and
   (iii) the change of the length of the mesh structure when expanding the endovascular device from a configuration when being at least partially accommodated within the distal portion of the placement device to the deployed configuration within the vasculature geometry; and/or
the image data includes at least one out of the group consisting of X-ray projection image data, a 3D reconstruction based on a plurality of X-ray projection images generated from different directions, a 3D computer tomography scan, a 3D magnet resonance tomography scan, a 3D model of at least part of the anatomical region of interest, and a combination thereof; and/or
interpreting the image data includes an identification of a wall apposition at the vascular wall of the part of the endovascular device being not accommodated within the placement device, and wherein estimating the position of the second end of the endovascular device after deployment is further based on the wall apposition of the already deployed part of the endovascular device at the vascular wall; and/or
estimating the position of the second end of the endovascular device after deployment is further based on the material properties of the endovascular device, the material properties include at least one out of the group consisting of elasticity, pretension, stiffness; and/or
the data processor is configured to determine instructions for positioning and deployment of the endovascular device including determining instructions for movement of the outer sheath and for movement of the inner shaft so that the endovascular device is positioned at a predetermined position in the vasculature and is deployed with a predetermined vasculature wall apposition; and/or
estimating the position of the second end of the endovascular device after deployment is further based on the determined instructions for positioning and deploying of the endovascular device; and/or
the data processor is configured to provide an output representing the determined instructions for positioning and deployment, including instructions executable by a robotic device performing the movements of the inner shaft and/or the movements of the outer sheath; and/or
artificial intelligence is utilized for at least one of generating the image data, interpreting the image data and estimating the position of the second end of the endovascular device after deployment; and/or
artificial intelligence is utilized for generating the 3D model of at least part of the anatomical region of interest; and/or
artificial intelligence is utilized for determining instructions for positioning and deployment of the endovascular device; and/or
the device further comprises a robotic device, wherein the robotic device comprises a first feature engaging the proximal end of the inner shaft and/or a second feature engaging the proximal end of the outer sheath so that the inner shaft and/or the outer sheath are moved in accordance with the determined instructions for positioning and deployment of the endovascular device, when the robotic device executes the determined instructions; and/or
the robotic device comprises at least one sensor providing information with respect to the amount of movement and the force applied when moving; and/or
the determined instructions for positioning and deployment of the endovascular device include at least one of the instructions out of the group consisting of (i) moving the inner shaft together with the outer sheath by pushing the inner shaft and the outer sheath distally so that the position of the endovascular device is changed relative to the vasculature, (ii) moving the inner shaft together with the outer sheath by pulling the inner shaft and the outer sheath proximally so that the position of the endovascular device is changed relative to the vasculature, (iii) moving the inner shaft together with the outer sheath by pushing the inner shaft and the outer sheath distally so that the diameter of at least a portion of the endovascular device is increased for vasculature wall apposition, (iv) moving the outer sheath and the inner shaft relative to each other so that the endovascular device is unsheathed, (v) moving the outer sheath and the inner shaft relative to each other so that the endovascular device is resheathed; and/or
the endovascular device is one out of the group consisting of a stent-like device, a flow-diverter with a mesh-type structure, a woven endobridge device, and an intra-cranial stent; and/or
the endovascular device is adapted to treat an aneurism; and/or
the endovascular device is a flow-diverter configured to be placed in an intra-cranial aneurism parent vessel; and/or
the device is configured to provide an alert when the estimated position of the second end of the endovascular device after deployment is outside of a predetermined area of the vasculature.

According to an aspect of the disclosure, a device is provided for assisting in placement of an endovascular device to be deployed for medical therapy, the device comprising a data processor configured to receive image data,
wherein the image data represent an anatomical region of interest including a vasculature, and at least a distal portion of a flexible elongated placement device together with the endovascular device located in the vasculature,
wherein the placement device comprises a distal end, a proximal end, a longitudinal direction extending between the distal end and the proximal end, an outer sheath extending along the longitudinal direction, and an inner shaft extending along the longitudinal direction, wherein the outer sheath and/or the inner shaft are movable one to the other in the longitudinal direction of the placement device,
wherein the endovascular device comprises a distal end, a proximal end, and an engagement feature for engagement at the distal end of the inner shaft of the placement device,
wherein the data processor is configured to interpret the image data and to determine instructions for positioning and deployment of the endovascular device while the endovascular device is in engagement with the inner shaft of the placement device,
wherein interpreting the image data comprises at least one of an identification of the position of the endovascular device relative to the vasculature and of an identification of a vasculature wall apposition of the endovascular device, and wherein determining instructions for positioning and deployment comprises determining instructions for movement of the outer sheath and for movement of the inner shaft so that the endovascular device is positioned at a predetermined position in the vasculature and is deployed with a predetermined vasculature wall apposition, and
wherein the data processor is further configured to provide an output representing the determined instructions for positioning and deployment; and/or
the determined instructions for positioning and deployment of the endovascular device include at least one of the instructions out of the group consisting of (i) moving the inner shaft together with the outer sheath by pushing the inner shaft and the outer sheath distally so that the position of the endovascular device is changed relative to the vasculature, (ii) moving the inner shaft together with the outer sheath by pulling the inner shaft and the outer sheath proximally so that the position of the endovascular device is changed relative to the vasculature, (iii) moving the inner shaft together with the outer sheath by pushing the inner shaft and the outer sheath distally so that the diameter of at least a portion of the endovascular device is increased for vasculature wall apposition, (iv) moving the outer sheath and the inner shaft relative to each other so that the endovascular device is unsheathed, (v) moving the outer sheath and the inner shaft relative to each other so that the endovascular device is resheathed; and/or
the output representing the determined instructions for positioning and deployment includes a visualization of instructions for moving of the inner shaft and of the outer sheath on a display device; and/or
the device further comprises a display, wherein the placement device comprises a handle engaging the proximal end of the inner shaft and engaging the proximal end of the outer sheath, so that the inner shaft and the outer sheath can be manually moved in accordance with the visualized instructions; and/or
   the output representing the determined instructions for positioning and deployment includes instructions executable by a robotic device performing the movements of the inner shaft and/or the movements of the outer sheath; and/or
the device further comprises a robotic device, wherein the robotic device comprises a first feature engaging the proximal end of the inner shaft and/or a second feature engaging the proximal end of the outer sheath so that the inner shaft and/or the outer sheath are moved in accordance with the determined instructions for positioning and deployment of the endovascular device, when the robotic device executes the determined instructions; and/or
the endovascular device is one out of the group consisting of a stent-like device, a flow-diverter with a mesh-type structure, a woven endobridge device, and an intra-cranial stent; and/or
the image data comprises X-ray image data; and/or
the endovascular device is adapted to treat an aneurism; and/or
the endovascular device is a flow-diverter configured to be placed in an intra-cranial aneurism parent vessel.

According to another aspect of the present disclosure, a method is provided for assisting in placement of an endovascular device, the method comprising the steps of
receiving image data,
interpreting the image data,
estimating a position of a second end of the endovascular device after deployment,
wherein the image data represent an anatomical region of interest including a vasculature, and at least a distal portion of a flexible elongated placement device together with the endovascular device located in the vasculature,
wherein the endovascular device is at least partially accommodated within the distal portion of the placement device and comprises a first end, a second end and a mesh structure allowing deployment of the endovascular device from a collapsed configuration for delivery to a deployed configuration, wherein the mesh structure has a length which changes during deployment,
wherein the placement device comprises a distal end, a proximal end, a longitudinal direction extending between the distal end and the proximal end, an outer sheath extending along the longitudinal direction, and an inner shaft extending along the longitudinal direction, wherein the outer sheath and/or the inner shaft are movable one to the other in the longitudinal direction of the placement device,
wherein interpreting of the image data includes an identification of the position of a first end of the endovascular device relative to the vasculature and an identification of a vasculature wall defining a vasculature geometry, and
wherein estimating a position of the second end of the endovascular device after deployment is based on the identified position of the first end of the endovascular device relative to the vasculature, on the vasculature geometry, and on a change of the length of the mesh structure when the endovascular device expands from a configuration when being at least partially accommodated within the distal portion of the placement device to the deployed configuration within the vasculature geometry; and/or
wherein interpreting the image data includes an identification of a wall apposition at the vascular wall of the part of the endovascular device being not accommodated within the placement device, and wherein estimating the position of the second end of the endovascular device after deployment is further based on the wall apposition of the already deployed part of the endovascular device at the vascular wall; and/or
the method comprising the step of determining instructions for positioning and deployment of the endovascular device including determining instructions for movement of the outer sheath and for movement of the inner shaft so that the endovascular device is positioned at a predetermined position in the vasculature and is deployed with a predetermined vasculature wall apposition; and/or
estimating the position of the second end of the endovascular device after deployment is further based on the determined instructions for positioning and deploying of the endovascular device; and/or
the method comprising the step of determining instructions for positioning and deployment, including instructions executable by a robotic device performing the movements of the inner shaft and/or the movements of the outer sheath; and/or
artificial intelligence is utilized for at least one out of the group consisting of generating the image data, interpreting the image data, estimating the position of the second end of the endovascular device after deployment, and generating a 3D model representing at least part of the anatomical region of interest; and/or
artificial intelligence is utilized for determining instructions for positioning and deployment of the endovascular device; and/or
the method comprising the step of providing an alert when the estimated position of the second end of the endovascular device after deployment is outside of a predetermined area of the vasculature.

According to another aspect of the disclosure, a method is provided for assisting in placement of an endovascular device, the method comprising the steps of
receiving image data,
interpreting the image data,
determining instructions for positioning and deployment of the endovascular device and providing an output representing the determined instructions,
wherein the image data represent an anatomical region of interest including a vasculature, and at least a distal portion of a flexible elongated placement device together with the endovascular device located in the vasculature,
wherein the placement device comprises a distal end, a proximal end, a longitudinal direction extending between the distal end and the proximal end, an outer sheath extending along the longitudinal direction, and an inner shaft extending along the longitudinal direction, wherein the outer sheath and/or the inner shaft are movable one to the other in the longitudinal direction of the placement device,
wherein the endovascular device comprises a distal end, a proximal end, and an engagement feature for engagement at the distal end of the inner shaft of the placement device,
wherein interpreting the image data comprises at least one of an identification of the position of the endovascular device relative to the vasculature and of an identification of a vasculature wall apposition of the endovascular device, and
wherein determining instructions for positioning and deployment comprises determining instructions for movement of the outer sheath and for movement of the inner shaft, while the endovascular device is in engagement with the inner shaft, so that the endovascular device is positioned at a predetermined position in the vessel and is deployed with a predetermined vasculature wall apposition; and/or
the determined instructions for positioning and deployment of the endovascular device include at least one of the instructions out of the group consisting of (i) moving the inner shaft together with the outer sheath by pushing the inner shaft and the outer sheath distally so that the position of the endovascular device is changed relative to the vasculature, (ii) moving the inner shaft together with the outer sheath by pulling the inner shaft and the outer sheath proximally so that the position of the endovascular device is changed relative to the vasculature, (iii) moving the inner shaft together with the outer sheath by pushing the inner shaft and the outer sheath distally so that the diameter of at least a portion of the endovascular device is increased for vasculature wall apposition, (iv) moving the inner shaft and the outer sheath relative to each other so that the endovascular device is unsheathed, (v) moving the inner shaft and the outer sheath relative to each other so that the endovascular device is resheathed; and/or
the movements are performed by a robotic device.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application.

However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims. For instance the skilled person can clearly use the assistance of placement of a diverter to be deployed for treatment of an aneurysm as above-described in details, to other medical vascular therapies than aneurysm treatment, by e.g. using a stent-like deployment instead of a diverter deployment, used to perform a medical therapy of a blood vascular disease or damage (e.g. local aneurysm, narrowed vessels due to occlusion due to clots, calcification..., vascular lesion ...).

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A system for assisting in deployment of a device to be deployed for medical therapy, wherein the device comprises a mesh structure allowing deployment of the device from a collapsed configuration to a deployed configuration, wherein the mesh structure has a shape which changes during deployment resulting in a change of a length of the device,
the system comprising a data processor configured to receive data, wherein the data relate to a position of a first point of the device and to the change of the length of the device, the device being at least partially in the deployed configuration,
wherein the data processor is configured to estimate a position of a second point of the device after deployment, based on
(i) the received data related to the position of the first point of the device, and
(ii) the received data related to the change of the length of the device.

2. The system of claim 1, wherein the device is an endovascular device and wherein the received data are further related to a vasculature, wherein the position of the first point is identified in relation to the vasculature, and wherein the position of the second point is estimated additionally based on the data related to the vasculature.

3. The system of claim 2, wherein the received data are image data representing an anatomical region of interest including the vasculature, and at least a distal portion of a flexible elongated placement device together with the endovascular device located in the vasculature.

4. The system of any of claims 2 to 3, wherein the processing unit is further configured to interpret the received data, the interpretation including an identification of a wall apposition at the vascular wall of the part of the endovascular device being not accommodated within the placement device, and wherein estimating the position of the second end of the endovascular device after deployment is further based on the wall apposition of the already deployed part of the endovascular device at the vascular wall.

5. The system of any of the previous claims, wherein the data processor is further configured to determine a length of a portion of the device being not accommodated within a distal portion of a flexible elongated placement device based on the received data.

6. The system of any of previous claims, wherein estimating the position of the second point of the endovascular device after deployment is further based on the material properties of the endovascular device, the material properties include at least one out of the group consisting of elasticity, pretension, stiffness.

7. The system of any of previous claims, wherein the placement device comprises a distal end, a proximal end, a longitudinal axis extending between the distal end and the proximal end, an outer sheath extending along the longitudinal axis, and an inner shaft extending along the longitudinal axis, wherein the outer sheath and/or the inner shaft are movable one to the other along the longitudinal axis of the placement device.

8. The system of claim 7, wherein the device is an endovascular device and wherein the data processor is further configured to determine instructions or parameters for positioning and deployment of the endovascular device taking into account the estimated position of the second point of the endovascular device, the determination including determining instructions for movement of the outer sheath and/or for movement of the inner shaft so that the endovascular device is positioned at a predetermined position in a vasculature and is deployed with a predetermined vasculature wall apposition.

9. The system of claim 8, wherein the determined instructions for positioning and deployment of the endovascular device include at least one of the instructions out of the group consisting of (i) moving the inner shaft together with the outer sheath by pushing the inner shaft and the outer sheath distally so that the position of the endovascular device is changed relative to the vasculature, (ii) moving the inner shaft together with the outer sheath by pulling the inner shaft and the outer sheath proximally so that the position of the endovascular device is changed relative to the vasculature, (iii) moving the inner shaft together with the outer sheath by pushing the inner shaft and the outer sheath distally so that the diameter of at least a portion of the endovascular device is increased for vasculature wall apposition, (iv) moving the inner shaft together with the outer sheath by pulling the inner shaft and the outer sheath proximally so that the diameter of at least a portion of the endovascular device is decreasing, (v) moving the outer sheath and the inner shaft relative to each other so that the endovascular device is unsheathed, (vi) moving the outer sheath and the inner shaft relative to each other so that the endovascular device is resheathed.

10. The system of claim 8 or 9, wherein the second point of the endovascular device is in an end portion of the endovascular device, wherein estimating the position of the second point of the endovascular device after deployment is further based on the determined instructions for positioning and deploying of the endovascular device.

11. The system of any of claims 8 to 10, wherein the data processor is configured to provide an output representing the determined instructions for positioning and deployment, including instructions executable by a robotic device performing the movements of the inner shaft and/or the movements of the outer sheath.

12. The system of claim 11, comprising the robotic device, wherein the robotic device comprises a first feature engaging the proximal end of the inner shaft and/or a second feature engaging the proximal end of the outer sheath so that the inner shaft and/or the outer sheath are moved in accordance with the determined instructions for positioning and deployment of the endovascular device, when the robotic device executes the determined instructions.

13. The system of claim 11 or 12, wherein estimating the position of the second point of the endovascular device after deployment is further based on data received from the robotic device related to the robotic action on the endovascular device.

14. The system of any of claims 7 to 13, wherein data processor is configured to estimate the position of the second point of the device after deployment, based further on data related to a phase of deployment identified from various predetermined phases of deployment of the device from the positioning device, such predetermined phases of deployment may include loading, unloading, unsheathing, re-sheathing.

15. A computer program product comprising processor-executable instructions which when executed on the data processor of the device according to claim 1,
the device comprising a mesh structure allowing deployment of the device from a collapsed configuration to a deployed configuration, wherein the mesh structure has a shape which changes during deployment resulting in a change of a length of the device,
the instructions causing the device to
receive data, wherein the data relate to a position of a first point of the device and to the change of the length of the device, the device being at least partially in the deployed configuration, and
estimate a position of a second point of the device after deployment, based on
(i) the received data related to the position of the first point of the device, and
(ii) the received data related to the change of the length of the device.
